# EUROPEAN PATENT SPECIFICATION

(11) **EP 1 311 494 B1**
(45) Date of publication and mention of the grant of the patent: **11.05.2005**
(21) Application number: 01967291.4
(22) Date of filing: 21.08.2001
(51) Int. Cl.: C07D 277/32, C07D 417/06

(54) **CONTINUOUS PROCESS FOR THE PREPARATION OF PESTICIDAL CHLOROTHIAZOLES**
KONTINUIERLICHES VERFAHREN ZUR HERSTELLUNG VON PESTIZIDEN CHLORTHIAZOLEN
PROCEDE CONTINU DE PREPARATION DE CHLOROTHIZAOLES PESTICIDES

(30) Priority: 23.08.2000 CH 165200
(43) Date of publication of application: 21.05.2003
(73) Proprietor: Syngenta Participations AG, 4058 Basel (CH)
(72) Inventor: FABER, Dominik, Syngenta Crop Protection, 4133 Schweizerhalle (CH); DESPONDS, Olivier, Syngenta Crop Prot Monthey SA, CH-1870 Monthey (CH); RAPOLD, Thomas, Syngenta Crop Protection, CH-4333 Münchwilen (CH); PASSAFARO, Marco, 8200 Schaffhausen (CH)
(74) Representative: Waterman, John Richard
(86) International application number: PCT/EP2001/009662
(87) International publication number: WO 2002/016334

(56) References cited:
- EP-A- 0 446 913
- EP-A- 1 031 566

## Description

A process for the preparation of a compound of formula 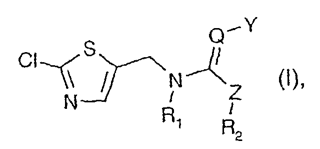 and, where appropriate, E/Z isomers, mixtures of E/Z isomers and/or tautomers thereof, in each case in free form or in salt form, wherein
- Q: is CH or N;
- Y: is NO₂ or CN;
- Z: is CHR₃, O, NR₃ or S;
- R₁ and R₂: either are each independently of the other hydrogen or C₁-C₈alkyl which is unsubstituted or substituted by R₄ or together are an alkylene bridge having two or three carbon atoms which optionally contains a hetero atom selected from the group consisting of NR₅, O and S,
- R₃: is H or C₁-C₁₂alkyl which is unsubstituted or substituted by R₄,
- R₄: is unsubstituted or substituted aryl or heteroaryl, and
- R₅: is H or C₁-C₁₂alkyl; wherein

a) a compound of formula 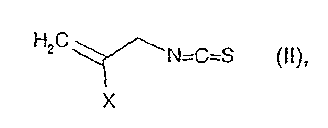 which is known or which can be prepared by known methods and wherein X is a leaving group, is reacted with a chlorinating agent to form a compound of formula 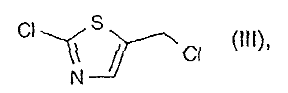 in free form or in salt form; and
b) the compound of formula (III) thereby obtained is reacted with a compound of formula 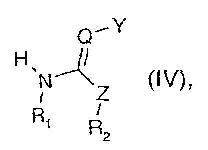 which is known or which can be prepared by methods known *per se* and wherein R₁, R₂, Y, Z and Q are as defined hereinbefore for the compound of formula (I);
in which process the chlorination according to process step a) is performed in a continuous process;
a process for the preparation of a compound of formula (III) according to process a) hereinbefore, and the use of compounds of formulae (II), (III) and (IV) in a process as described hereinbefore.

The compounds of formula (I) are known as valuable pesticides, and synthesis methods for those compounds are described in the literature. It has, however, been found that significant safety problems occur in the case of those processes known from the literature. It has moreover been found that the compound of formula (III) prepared according to the known processes does not satisfy the requirements in terms of purity either, that it is - probably because of those impurities - thermally unstable, which in turn can lead to significant problems in a production plant, and also that the known processes have significant disadvantages in respect of further parameters, for example yield, duration of the synthesis cycle, volume yield, disposal of ecologically and toxicologically problematic wastes, for example solvents.

The known preparation processes are therefore not satisfactory in every respect, which is why there is a need to provide improved preparation processes for the compounds of formula (I) and especially of formula (III).
It is mentioned in EP-A-446 913, Example 1, that when the compound of formula 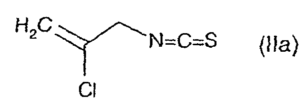 is chlorinated in chloroform there is first formed a mixture of intermediates, the structures of which are postulated as being 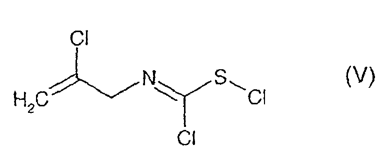 and

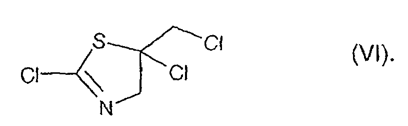

According to EP-A-446 913, that mixture of intermediates is allowed to react completely, with cooling at about +40°C, to form the compound of formula (III). It has now been found that, in the case of that procedure, a dangerous heating potential, *inter alia*, is built up, which in an unfavourable case may result in a major incident. Using the process according to the invention, that problem is avoided by carrying out the reaction continuously, only small amounts of the said intermediates being accumulated per unit of time and, moreover, the residence times in the individual reactors being short. When required, catalysts are added to the reaction mixture during that continuous procedure, the nature and amount of the catalyst and the time of addition being dependent upon the other conditions of the reaction procedure.

In order to avoid an accumulation of intermediates such as the compounds (V) and (VI) and inadequate selectivity of the reaction procedure in a large vessel in which the process is carried out batch-wise, the following possibilities, *inter alia*, exist:

The reaction step leading from the postulated intermediate (V) to intermediate (VI) has an especially great heating effect, so that accumulation of the compound having the presumed structure (V) should if possible be avoided. It has now been found that, in a preferred embodiment, a catalysed reaction can be performed which proceeds from the above compound of formula (II) directly - that is, without significant accumulation of the compound of formula (V) - to an intermediate that probably corresponds to formula (VI) above. That catalysed reaction is carried out especially at from -30°C to +50°C, more especially at from -20°C to +30°C, very especially at from -10°C to +20°C. The reaction is catalysed, for example, by SO₂, SO₂Cl₂, polar solvents, e.g. acetonitrile or nitromethane, and the compound of formula (III) itself, metals, e.g. Hastelloy, and metals salts, e.g. FeCl₃, and by combinations of such catalysts, e.g. SO₂/acetonitrile.

The conversion of the presumed compound (VI) into compound (III) is in turn catalysed by polar solvents, e.g. acetonitrile or nitromethane, and 2-chloro-5-chloromethyl-thiazole of formula (III), HCl, metals, e.g. Hastelloy, and metals salts, e.g. FeCl₃, and by combinations of such catalysts, e.g. compound (III) in the presence of HCl. The reaction is carried out at from +30°C to +80°C, especially at from +40°C to +60°C, more especially at from +45°C to +55°C.

Another, likewise preferred embodiment is the continuous preparation of the compound of formula (III) *via* the preparation of the presumed high-energy intermediate (V) at from -30°C to +30°C, preferably at from -20°C to +20°C, especially at from -10°C to 10°C, followed by continuous passing-over onto previously prepared compound (III), which itself acts catalytically. In order to prevent high thermal accumulation in the form of the compound for which structure (V) is postulated, the reaction of compound (V) to form compound (III) must in this case be catalysed. That reaction is catalysed by SO₂, SO₂Cl₂, polar solvents, e.g. acetonitrile or nitromethane, and compound (III), HCl, metals, e.g. Hastelloy, and metals salts, e.g. FeCl₃, and by combinations of such catalysts, e.g. SO₂ in the presence of acetonitrile or 2-chloro-5-chloromethyl-thiazole in the presence of HCl. That reaction step is carried out at from +30°C to 80°C, preferably at from +40°C to 60°C, especially at from +45°C to +55°C.

An especially preferred aspect according to the invention is a solventless procedure. In this procedure, preference is given to a temperature range in a first process step of from -30°C to +30°C, especially from -10°C to +10°C; and in a second process step of from +30°C to +80°C, preferably from +45°C to +60°C. A preferred embodiment consists of performing one or both reaction steps, which presumably proceed by way of the compounds of formulae (V) and (VI) mentioned hereinbefore, in the presence of 2-chloro-5-chloromethylthiazole.

In a continuous process carried out without solvents, that is to say in the melt, an especially high production capacity can be achieved with a high degree of process safety. Because the reaction mixture rapidly becomes solid because of the comparatively high melting point of the product, solventless process variants cannot be carried out batch-wise at low temperatures.

Some compounds of formulae (I) to (IV) contain asymmetric carbon atoms, as a result of which the compounds may occur in optically active forms. Formulae (I) to (IV) are intended to include all those possible isomeric forms, and mixtures thereof, for example racemates or mixtures of E/Z isomers.

The general terms used hereinbefore and hereinafter have the following meanings, unless defined otherwise:

Unless otherwise defined, carbon-containing groups and compounds each contain from 1 up to and including 8, preferably from 1 up to and including 6, especially from 1 up to and including 4, and more especially 1 or 2, carbon atoms.

Alkyl, both as a group *per se* and as a structural element of other groups and compounds, for example haloalkyl, arylalkyl and hydroxyalkyl, is, in each case taking due account of the particular number of carbon atoms contained in the group or compound in question, either straight-chained, i.e. methyl, ethyl, propyl, butyl, pentyl or hexyl, or branched, e.g. isopropyl, isobutyl, sec-butyl, tert-butyl, isopentyl, neopentyl or isohexyl.

Alkenyl, both as a group *per se* and as a structural element of other groups and compounds, for example haloalkenyl and arylalkenyl, is, in each case taking due account of the particular number of carbon atoms contained in the group or compound in question, either straight-chained, for example vinyl, 1-methylvinyl, allyl, 1-butenyl or 2-hexenyl, or branched, for example isopropenyl.

Alkynyl, both as a group *per se* and as a structural element of other groups and compounds, for example haloalkynyl, is, in each case taking due account of the particular number of carbon atoms contained in the group or compound in question, either straight-chained, for example propargyl, 2-butynyl or 5-hexynyl, or branched, for example 2-ethynyl-propyl or 2-propargylisopropyl.

C₃-C₆Cycloalkyl is cyclopropyl, cyclobutyl, cyclopentyl or cyclohexyl, especially cyclohexyl.

Aryl is phenyl or naphthyl, especially phenyl.

Heteroaryl is to be understood as being a five- to seven-membered monocyclic aromatic ring having from one to three hetero atoms selected from the group consisting of N, O and S, especially N and S, or a bicyclic heteroaryl that may contain either in only one ring - as, for example, in quinolinyl, quinoxalinyl, indolinyl, benzothiophenyl or benzofuranyl - or in both rings - as, for example, in pteridinyl or purinyl - independently of one another one or more hetero atoms selected from N, O and S. Preference is given to pyridyl, pyrimidinyl, thiazolyl and benzothiazolyl.

Halogen, both as a group *per se* and as a structural element of other groups and compounds, for example haloalkyl, haloalkenyl and haloalkynyl, is fluorine, chlorine, bromine or iodine, especially fluorine, chlorine or bromine, more especially chlorine or bromine, and very especially chlorine.

Halo-substituted, carbon-containing groups and compounds, for example haloalkyl and haloalkenyl, may be partially halogenated or perhalogenated, the halogen substituents in the case of multiple halogenation being identical or different. Examples of haloalkyl, both as a group *per se* and as a structural element of other groups and compounds, for example haloalkenyl, are methyl mono- to tri-substituted by fluorine, chlorine and/or by bromine, for example CHF₂ or CF₃; ethyl mono- to penta-substituted by fluorine, chlorine and/or by bromine, for example CH₂CF₃, CF₂CF₃, CF₂CCl₃, CF₂CHCl₂, CF₂CHF₂, CF₂CFCl₂, CF₂CHBr₂, CF₂CHClF, CF₂CHBrF or CClFCHClF; propyl or isopropyl each mono- to hepta-substituted by fluorine, chlorine and/or by bromine, for example CH₂CHBrCH₂Br, CF₂CHFCF₃, CH₂CF₂CF₃ or CH(CF₃)₂; and butyl, or one of the isomers thereof, mono- to nona-substituted by fluorine, chlorine and/or by bromine, for example CF(CF₃)CHFCF₃ or CH₂(CF₂)₂CF₃. Haloalkenyl is, for example, CH₂CH=CHCl, CH₂CH=CCl₂, CH₂CF=CF₂ or CH₂CH=CHCH₂Br.

A leaving group X is to be understood hereinbefore and hereinafter as being any removable group customarily considered for chemical reactions, as will be known to the person skilled in the art, especially a halogen such as fluorine, chlorine, bromine or iodine, -O-C(=O)-A, -O-P(=O)(-A)₂, -O-Si(C₁-C₈alkyl)₃, -O-(C₁-C₈alkyl), -O-aryl, -O-S(=O)₂A, -S-P(=O)(-A)₂, -S-P(=S)(-A)₂, -S-(C₁-C₈alkyl), -S-aryl, -S(=O)A, -S(=O)₂A or -O-C(=O)-A wherein A is unsubstituted or substituted C₁-C₈alkyl, C₂-C₈alkenyl or C₂-C₈alkynyl, unsubstituted or substituted aryl, unsubstituted or substituted benzyl, C₁-C₈alkoxy or di(C₁-C₈alkyl)amine wherein the alkyl groups are independent of one another; NO₃, NO₂, or sulfate, sulfite, phosphate, phosphite, carboxylate, imino ester, N₂ or carbamate.

Some compounds of formulae (I) to (IV) may be in the form of tautomers. Those compounds are therefore to be understood hereinbefore and hereinafter as including corresponding tautomers, even if the latter are not specifically mentioned in each case.

Compounds of formulae (I) to (IV) having at least one basic centre are capable, for example, of forming acid addition salts. Those acid addition salts are formed, for example, with strong inorganic acids, such as mineral acids, e.g. perchloric acid, sulfuric acid, nitric acid, nitrous acid, a phosphoric acid or a hydrohalic acid, with strong organic carboxylic acids, such as unsubstituted or substituted, e.g. halo-substituted, C₁-C₄alkanecarboxylic acids, e.g. acetic acid, saturated or unsaturated dicarboxylic acids, e.g. oxalic, malonic, succinic, maleic, fumaric and phthalic acid, hydroxycarboxylic acids, e.g. ascorbic, lactic, malic, tartaric and citric acid, or benzoic acid, or with organic sulfonic acids, such as unsubstituted or substituted, e.g. halo-substituted, C₁-C₄alkane- or aryl-sulfonic acids, e.g. methane- or p-toluene-sulfonic acid. Furthermore, compounds of formulae (I) to (IV) having at least one acid group are capable of forming salts with bases. Suitable salts with bases are, for example, metal salts, such as alkali metal and alkaline earth metal salts, e.g. sodium, potassium and magnesium salts, and salts with ammonia or an organic amine, such as morpholine, piperidine, pyrrolidine, a mono-, di- or tri-lower alkylamine, e.g. ethyl-, diethyl-, triethyl- or dimethyl-propyl-amine, or a mono-, di- or tri-hydroxy-lower alkylamine, e.g. mono-, di- or tri-eihanofamine. In addition, corresponding internal salts may optionally also be formed. The compounds of formulae (I) to (IV) are to be understood hereinbefore and hereinafter as including both the compounds of formulae (I) to (IV) in free form and the corresponding salts. The same is correspondingly true for tautomers of compounds of formulae (I) to (IV) and salts thereof. In the case of the compounds of formulae (I) and (III), preference is generally given in each case to a process for the preparation of the free form.

Preference is given within the scope of the invention to a process for the preparation of a compound of formula (I)
(1) wherein R₁ and R₂ in the compounds of formulae (I) and (IV) either are each independently of the other hydrogen or C₁-C₄alkyl or together are a two- or three-membered alkylene bridge which optionally contains a hetero atom from the group consisting of NR₅, O and S, and R₅ is H or C₁-C₄alkyl;
   and especially are hydrogen or together are a two- or three-membered alkylene bridge which optionally contains a hetero atom from the group consisting of NR₅ and O, and R₅ is C₁-C₄alkyl;
   and more especially R₁ and R₂ together are -CH₂-O-CH₂-, -CH₂-CH₂-CH₂- or -CH₂-CH₂-;
(2) wherein Q is N;
(3) wherein Y is NO₂;
(4) wherein Z is NR₃ and R₃ is H or C₁-C₄alkyl;
(5) wherein X in the compound of formula (II) is halogen such as fluorine, chlorine, bromine or iodine, -O-C(=O)-A, -O-P(=O)(-A)₂, -O-S(=O)₂A, -S-P(=O)(-A)₂, -S-P(=S)(-A)₂, -S(=O)A or -S(=O)₂A, wherein A is unsubstituted or substituted C₁-C₈alkyl, C₂-C₈alkenyl or C₂-C₈alkynyl, unsubstituted or substituted aryl, unsubstituted or substituted benzyl, C₁-C₈alkoxy or di(C₁-C₈alkyl)amine wherein the alkyl groups are independent of one another; especially wherein X is chlorine, bromine or iodine, more especially chlorine or bromine; very especially wherein X is chlorine;
(6) wherein, in process step a), for the purpose of chlorination, SO₂ is used as catalyst in an amount of from 1 mol % to 50 mol %, preferably from 10 mol % to 40 mol %, especially from 15 mol % to 30 mol %, based on the starting material of formula (II);
(7) wherein, in process step a), SO₂ is used in the form of SO₂ gas or in the form of an SO₂-releasing agent, preferably SO₂Cl₂;
(8) wherein the continuous process is performed in the absence of a solvent;
(9) wherein the continuous process is performed in the presence of 2-chloro-5-chloromethyl-thiazole;
(10) wherein the continuous process is performed under reduced pressure, preferably at 50 to 500 mbar, more especially at 50 to 200 mbar; very especially at 80 to 120 mbar.

Especially suitable process conditions can be found in the Examples.

The process is especially suitable for the preparation of thiamethoxam, known from WO 98/32747; and of Ti-435 (clothianidin), known from EP-A-446 913.

### Process step a):

The reaction of process step a) described hereinbefore and hereinafter is carried out, if necessary, in a closed vessel, under pressure, in an inert gas atmosphere and/or under anhydrous conditions. Especially advantageous reaction conditions can be found in the Examples.

The reactants can be reacted with one another as such, i.e. without a solvent or diluent, for example in molten form. In some cases, however, the addition of a solvent or diluent is advantageous. Suitable solvents include aprotic solvents, especially, for example: aliphatic, aromatic and alicyclic hydrocarbons, such as benzene, toluene, xylene, mesitylene, Tetralin, chlorobenzene, dichlorobenzene, bromobenzene, petroleum ether, hexane, cyclohexane, dichloromethane, trichloromethane, carbon tetrachloride, dichloroethane, trichloroethene and tetrachloroethene; esters, such as ethyl acetate, methyl acetate, dimethyl carbonate, diethyl carbonate, methyl formate, ethyl formate, ethoxyethyl acetate and methoxyethyl acetate; ethers, such as diethyl ether, dipropyl ether, diisopropyl ether, dibutyl ether, tert-butyl methyl ether, ethylene glycol dimethyl ether, dimethoxydiethyl ether, tetrahydrofuran and dioxane; ketones, such as acetone, methyl ethyl ketone, methyl isopropyl ketone and methyl isobutyl ketone; amides, such as N,N-dimethylformamide, N,N-diethylformamide, N,N-dimethylacetamide, N-methylpyrrolidone and hexamethylphosphoric acid triamide; nitriles, such as acetonitrile and propionitrile; and sulfoxides, such as dimethyl sulfoxide; nitroalkanes and aromatic nitro compounds, such as nitromethane, nitroethane and nitrobenzene; or mixtures of such solvents. Preference is given to polar aprotic solvents, especially carboxylic acid derivatives such as amides and nitriles; especially preferred solvents can be found in the Examples.

Suitable chlorinating agents include especially Cl₂, SO₂Cl₂, POCl₃, PCl₃ and PCl₅; or mixtures thereof; especially Cl₂.

Catalytic amounts are to be understood as less-than-stoichiometric amounts based on the starting material of formula (II). SO₂ may either be added as such in gaseous form, or a compound capable of releasing SO₂ may be added. SO₂Cl₂ is especially suitable for that purpose.

### Process step b):

The reactants can be reacted with one another as such, i.e. without the addition of a solvent or diluent, for example in molten form. In most cases, however, the addition of an inert solvent or diluent, or a mixture thereof, is advantageous. Examples of such solvents or diluents that may be mentioned are more or less the same as those mentioned under process step a), although in addition protic solvents, such as alcohols and protic amides, are also suitable. When the reaction in question is carried out in the presence of a base, bases that are used in excess, such as triethylamine, pyridine, N-methylmorpholine or N,N-diethyl-aniline, may also serve as solvents or diluents.

The reaction is carried out preferably at a temperature of from approximately 0°C to approximately +180°C, especially from about +10°C to about +80°C, in many cases between room temperature and the reflux temperature of the solvent. In an especially preferred embodiment of process step b), a compound of formula (IV) is reacted at from 0°C to 120°C, especially from 20°C to 80°C, preferably from 30°C to 60°C, in an ester, especially in dimethyl carbonate, and preferably in the presence of a base, especially K₂CO₃.

The reaction is preferably carried out at normal pressure.

The reaction time is not critical; preference is given to a reaction time of from 0.1 to 48 hours, especially from 0.5 to 12 hours.

The product is isolated by the usual methods, for example by filtration, crystallisation, distillation or chromatography or any suitable combination of such methods.

The yields achieved are usually good. It is often possible to obtain a yield of 80 % of the theoretical value.

Preferred conditions under which the reaction is carried out can be found in the Examples.

Salts of compounds of formulae (I) to (IV) can be prepared in a manner known *per se.* For example, acid addition salts are obtained by treatment with a suitable acid or a suitable ion exchange reagent and salts with bases by treatment with a suitable base or a suitable ion exchange reagent.

Salts of compounds of formulae (I) to (IV) can be converted into the free compounds of formulae (I) to (IV) in customary manner; acid addition salts can be converted, for example, by treatment with a suitable basic medium or a suitable ion exchange reagent and salts with bases, for example, by treatment with a suitable acid or a suitable ion exchange reagent.

Salts of compounds of formulae (I) to (IV) can be converted into different salts of compounds of formulae (I) to (IV) in a manner known *per se*; for example, acid addition salts can be converted into different acid addition salts, for example by treatment of a salt of an inorganic acid, such as a hydrochloride, with a suitable metal salt, such as a sodium, barium or silver salt, of an acid, for example with silver acetate, in a suitable solvent in which an inorganic salt being formed, for example silver chloride, is insoluble and is therefore precipitated out from the reaction mixture.

Depending upon the procedure and/or the reaction conditions, the compounds of formulae (I) to (IV) having salt-forming properties can be obtained in free form or in the form of salts.

The compounds of formulae (I) to (IV) and, in each case, where applicable, the tautomers thereof, in each case in free form or in salt form, may be in the form of one of the possible isomers or in the form of a mixture thereof, for example depending upon the number of asymmetric carbon atoms occurring in the molecule and the absolute and relative configuration thereof and/or depending upon the configuration of non-aromatic double bonds occurring in the molecule, in the form of pure isomers, such as antipodes and/or diastereoisomers, or in the form of mixtures of isomers, such as mixtures of enantiomers, for example racemates, mixtures of diastereoisomers or mixtures of racemates; the invention relates both to the pure isomers and to all possible mixtures of isomers and this is to be understood accordingly hereinbefore and hereinafter, even when stereochemical details are not specifically mentioned in each case.

Mixtures of diastereoisomers or mixtures of racemates of compounds of formulae (I) to (IV), or salts thereof, obtainable in accordance with the process - depending upon the starting materials and procedures chosen ― or by other means can be separated into the pure diastereoisomers or racemates in known manner on the basis of the physico-chemical differences between the constituents, for example by fractional crystallisation, distillation and/or chromatography.

Mixtures of enantiomers, such as racemates, so obtainable can be separated into the optical antipodes by known methods, for example by recrystallisation from an optically active solvent, by chromatography on chiral adsorbents, for example high-pressure liquid chromatography (HPLC) on acetyl cellulose, with the aid of suitable microorganisms, by cleavage with specific immobilised enzymes, via the formation of inclusion compounds, for example using chiral crown ethers, in which case only one enantiomer is complexed, or by conversion into diastereoisomeric salts, for example by reacting a basic end product racemate with an optically active acid, such as a carboxylic acid, for example camphoric acid, tartaric acid or malic acid, or a sulfonic acid, for example camphorsulfonic acid, and separating the mixture of diastereoisomers obtainable in that manner, for example on the basis of their different solubilities by fractional crystallisation, into the diastereoisomers, from which the desired enantiomer can be freed by the action of suitable, for example basic, media.

Pure diastereoisomers and enantiomers can be obtained not only by separation of corresponding mixtures of isomers but also, according to the invention, by generally known methods of diastereoselective or enantioselective synthesis, for example by carrying out the process according to the invention with starting materials that have appropriate stereochemistry.

The compounds of formulae (I) to (IV), and salts thereof, may also be obtained in the form of hydrates and/or may include other solvents, for example solvents that may optionally have been used for the crystallisation of compounds that occur in solid form.

The invention relates to all those embodiments of the process according to which a compound obtainable as starting material or intermediate at any stage of the process is used as starting material and all or some of the remaining steps are carried out, or in which a starting material is used in the form of a derivative or a salt and/or its racemates or antipodes, or, especially, is formed under the reaction conditions.

Compounds of formulae (I), (III) and (IV) obtainable in accordance with the process or by other means can be converted into different corresponding compounds in a manner known *per se*.

In the processes of the present invention there are preferably used those starting materials and intermediates, in each case in free form or in salt form, which result in the compounds of formula (I) or salts thereof described at the beginning as being especially valuable.

The invention relates especially to the preparation processes described in the Examples.

The present invention relates also to the process for the preparation of a compound of formula (III) from a compound of formula (II) according to process step a) as defined hereinbefore.

The preferences applying to the substituents of compounds of formula (IV) are the same as those mentioned hereinbefore in the processes for the preparation of compounds of formula (I).

The compounds of formulae (II) and (IV) are known, for example as intermediates for the preparation of pesticides, or they can be prepared using processes known *per se*.

### Preparation Examples

### A) Preparation of 2-chloro-5-chloromethyl-thiazole

Example P1: At from 20°C to 22°C, 925 g of a solution containing 33 % (by weight) 2-chloro-3-isothiocyanato-1-propene in acetonitrile, 177.5 g of chlorine and 24.7 g of SO₂ are continuously and simultaneously introduced, per hour, into a loop reactor having a volume of 370 ml. The reaction mixture flows over into an after-reactor having an internal temperature of 50°C. Each hour there are obtained from the after-reactor 1086 g of a reaction solution containing 32.2 % 2-chloro-5-chloromethyl-thiazole by weight, which corresponds to a yield of 91.1 % of theory, based on 2-chloro-3-isothiocyanato-1-propene.

Example P2: (continuous reaction procedure, without solvents): chlorination of 2-chloro-3-isothiocyanato-1-propene using SO₂Cl₂ in 2-chloro-5-chloromethyl-thiazole

1.05 molar equivalents of SO₂Cl₂ and one molar equivalent of 2-chloro-3-isothiocyanato-1-propene as a 50 % solution in 2-chloro-5-chloromethyl-thiazole are metered into a through-flow reactor in parallel in such a manner that the internal temperature can be maintained at from 10 to 20°C for a residence time of from 20 to 30 minutes. The reaction mixture is continuously passed over onto a melt of 2-chloro-5-chloromethyl-thiazole at 50°C which is located in a second reactor. In that second vessel, the reaction gases (SO₂/HCl) are driven off in controlled metered amounts. Conversion into 2-chloro-5-chloromethyl-thiazole in the second cascade vessel is about 97 % for a residence time of one hour. In a third vessel, conversion into 2-chloro-5-chloromethyl-thiazole is completed and the residual HCl is driven off. Gas chromatographic analysis of the crude product shows a yield of 92 % of theory, based on 2-chloro-3-isothiocyanato-1-propene.

Example P3: 1.05 molar equivalents of Cl₂ and one molar equivalent of 2-chloro-3-isothiocyanato-1-propene are metered into a through-flow reactor in parallel in such a manner that the internal temperature can be maintained at from -30 to 0°C. The reaction mixture is continuously passed over into a second reactor containing a mixture of 2-chloro-5-chloromethyl-thiazole and 2 mol % FeCl₃. In that second vessel, the reaction gas (HCl) is driven off in controlled metered amounts. Conversion into 2-chloro-5-chloromethyl-thiazole in the second cascade vessel is about 98 % for a residence time of one hour. In a third vessel, conversion into 2-chloro-5-chloromethyl-thiazole is completed and the residual HCl is driven off. Gas chromatographic analysis of the crude product shows a yield of 84 % of theory, based on 2-chloro-3-isothiocyanato-1-propene.

Example P4: 0.9 molar equivalent of Cl₂ and one molar equivalent of 2-chloro-3-isothiocyanato-1-propene, together with a catalytic amount of 0.15 molar equivalent of SO₂Cl₂ in the presence of a Hastelloy probe, are metered into a through-flow reactor in parallel in such a manner that the internal temperature can be maintained at from -5 to 5°C for a residence time of from 10 to 20 minutes. The reaction mixture is continuously passed over onto a melt of 2-chloro-5-chloromethyl-thiazole at 50°C. In the second vessel, the reaction gases (SO₂/HCl) are, to a large extent, driven off in controlled metered amounts. Conversion into 2-chloro-5-chloromethyl-thiazole in the second cascade vessel is about 95 % for a residence time of one hour. In a third vessel, conversion into 2-chloro-5-chloromethyl-thiazole is completed and the residual HCl is driven off. Gas chromatographic analysis of the crude product shows a yield of 76 % of theory.

Example P5: 500 g 2-chloro-5-chloromethyl-thiazole are charged at 40 to 42 °C and 100 mbar to a cascade reactor. 145 g 2-chloro-3-isothiocyanato-1-propene and 90 g chlorine per hour are simultaneously and continously introduced. The reaction mixture is allowed to flow over into a second reactor having an internal temerature of 50 °C. The yield per hour in this process is 183 g of crude melt containing 78 % of 2-chloro-5-chloromethyl-thiazole.

### B) Preparation of 3-(2-chloro-thiazol-5-yl-methyl)-5-methyl-4-nitroimino-perhydro-1,3,5-oxadiazine

Example P6: 184 g of 3-methyl-4-nitroimino-perhydro-1,3,5-oxadiazine 100 % are introduced into 400 g of dimethyl carbonate in a sulfonation flask and 168 g of 2-chloro-5-chloromethylthiazole 100 % are added. The mixture is heated to 65°C. With stirring at from 62 to 68°C, a mixture consisting of 350 g of dimethyl carbonate, 4 g of tetramethylammonium hydroxide pentahydrate and 242 g of potassium carbonate powder is metered in over the course of 60 minutes, thorough stirring of the reaction mixture being maintained until more than 99 % of the 2-chloro-5-chloromethylthiazole has been converted.

The reaction mixture is then cooled and 600 g of water are added. The pH is adjusted to 6.5 using about 260 g of 32 % hydrochloric acid; the mixture is left to stand until the phases have separated, and the organic phase is separated off. The organic phase is concentrated at 60°C *in vacuo* to a final weight of 600 g. The mixture is slowly cooled to 0-5°C, which is maintained for one hour. The resulting suspension is then filtered.

218 g of the title product having a purity of from 98 to 99 % (74 % of theory, based on 2-chloro-5-chloromethylthiazole 100 %) are obtained.

## Claims

1. A process for the preparation of a compound of formula 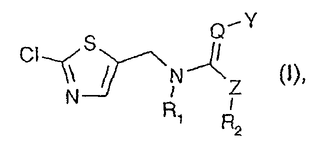 and, where appropriate an E/Z isomer, a mixture of E/Z isomers and/or a tautomer thereof, in each case in free form or in salt form, wherein
Q is CH or N;
Y is NO₂ or CN;
Z is CHR₃, O, NR₃ or S;
R₁ and R₂ either are each independently of the other hydrogen or C₁-C₈alkyl which is unsubstituted or substituted by R₄ or together are an alkylene bridge having two or three carbon atoms which optionally contains a hetero atom selected from the group consisting of NR₅, O and S,
R₃ is H or C₁-C₁₂alkyl which is unsubstituted or substituted by R₄,
R₄ is unsubstituted or substituted aryl or heteroaryl, and
R₅ is H or C₁-C₁₂alkyl; wherein
a) a compound of formula 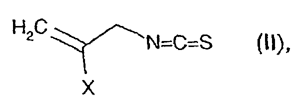 which is known or which can be prepared by known methods and wherein X is a leaving group, is reacted with a chlorinating agent to form a compound of formula 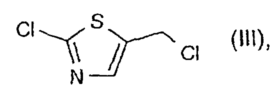 in free form or in salt form; and
b) the compound of formula (III) thereby obtained is reacted with a compound of formula 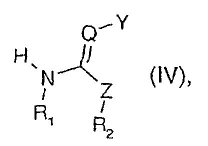 which is known or which can be prepared by methods known *per se* and wherein R₁, R₂, Y, Z and Q are as defined hereinbefore for the compound of formula (I);
in which process the chlorination according to process step a) is performed in a continuous process.

2. A process according to claim 1, wherein in process step a) a polar aprotic solvent is used.

3. A process according to claim 1, which is carried out without solvents.

4. A process according to any one of claims 1 to 3, which is carried out in the presence of previously manufactured 2-chloro-5-chloromethyl-thiazole.

5. A process for the preparation of a compound of formula 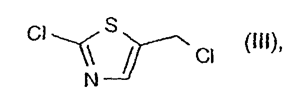 wherein a compound of formula 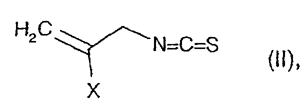 wherein X is a leaving group, in free form or in salt form, is reacted with a chlorinating agent;
in which process the reaction is performed in a continuous process.

## Patentansprüche

1. Verfahren zur Herstellung einer Verbindung der Formel 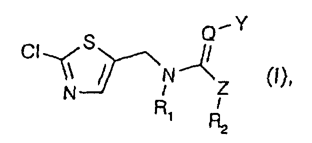 und, sofern gegeben, eines E/Z-Isomeren, einer Mischung von E/Z-Isomeren und/oder eines Tautomeren hiervon in jedem Fall in freier Form oder in Salzform, worin
Q CH oder N darstellt;
Y NO₂ oder CN darstellt;
Z CHR₃, O, NR₃ oder S darstellt;
R₁ und R₂ jeweils unabhängig voneinander Wasserstoff oder C₁-C₈-Alkyl darstellen, das unsubstituiert oder durch R₄ substituiert ist oder gemeinsam eine Alkylenbrücke, die zwei oder drei Kohlenstoffatome aufweist, die gegebenenfalls ein Heteroatom enthalten ausgewählt aus der Gruppe bestehend aus NR₅, O und S, darstellen;
R₃ H oder C₁-C₁₂-Alkyl, das unsubstituiert oder durch R₄ substituiert ist, darstellt,
R₄ unsubstituiertes oder substituiertes Aryl oder Heteroaryl darstellt, und
R₅ H oder C₁-C₁₂-Alkyl darstellt; worin
a) eine Verbindung der Formel 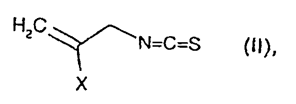 die bekannt ist oder durch bekannte Methoden hergestellt werden kann, und worin X eine Abgangsgruppe darstellt, mit einem Chlorierungsmittel zur Reaktion gebracht wird, um eine Verbindung der Formel 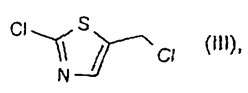 in freier oder in Salzform zu bilden; und
b) die dadurch erhaltene Verbindung der Formel (III) mit einer Verbindung der Formel 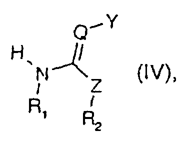 zur Reaktion gebracht wird, die bekannt ist oder die nach an sich bekannten Methoden hergestellt werden kann und worin R₁, R₂, Y, Z und Q wie vorstehend für die Verbindung der Formel (I) definiert sind;
in welchem Verfahren die Chlorierung gemäß Verfahrensstufe a) in einem kontinuierlichen Verfahren ausgeführt wird.

2. Verfahren gemäß Anspruch 1, worin in Verfahrensstufe a) ein polares aprotisches Lösungsmittel verwendet wird.

3. Verfahren gemäß Anspruch 1, das ohne Lösungsmittel ausgeführt wird.

4. Verfahren gemäß den Ansprüchen 1 bis 3, das in Anwesenheit von zuvor hergestelltem 2-Chlor-5-chlormethyl-thiazol ausgeführt wird.

5. Verfahren zur Herstellung einer Verbindung der Formel 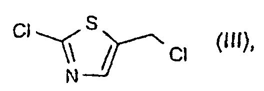 worin eine Verbindung der Formel 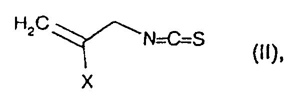 worin X eine Abgangsgruppe darstellt, in freier Form oder in Salzform mit einem Chlorierungsmittel zur Reaktion gebracht wird,
in welchem Verfahren die Reaktion als ein kontinuierliches Verfahren ausgeführt wird.

## Revendications

1. Procédé de préparation d'un composé de formule 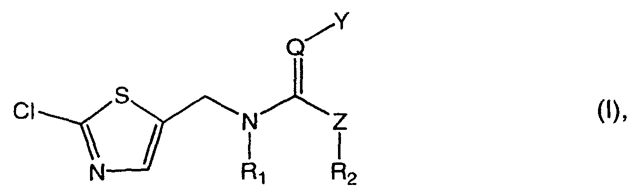 et, si approprié, un isomère E/Z, un mélange d'isomères E/Z et/ou un tautomère de celui-ci, dans chaque cas sous forme libre ou sous forme de sel, dans lequel
Q est CH ou N ;
Y est NO₂ ou CN ;
Z est CHR₃, O, NR₃ ou S ;
R₁ et R₂ soit sont chacun indépendamment de l'autre l'hydrogène soit un groupe alkyle en C₁-C₈ qui est non substitué ou substitué par R₄ ou ensemble sont un pont alkylène ayant deux ou trois atomes de carbone qui contiennent éventuellement un hétéroatome choisi dans le groupe constitué par NR₅, O et S ;
R₃ est H ou un groupe alkyle en C₁-C₁₂ qui est non substitué ou substitué par R₄ ;
R₄ est un groupe aryle ou hétéroaryle non substitué ou substitué ; et
R₅ est H ou un groupe alkyle en C₁-C₁₂ ; dans lequel
a) on fait réagir un composé de formule 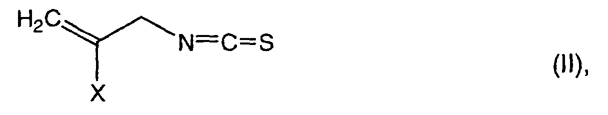 qui est connu ou qui peut être préparé par des méthodes connues et dans lequel X est un groupe labile, avec un agent de chloration pour former un composé de formule 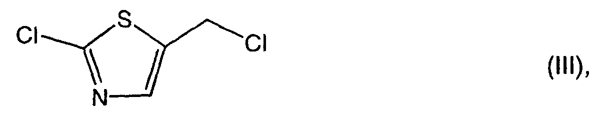 sous forme libre ou sous forme de sel ; et
b) on fait réagir le composé de formule (III) ainsi obtenu avec un composé de formule 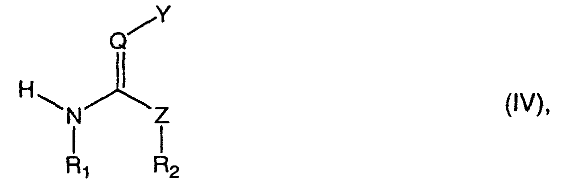 qui est connu ou qui peut être préparé par des méthodes connues *per se* et dans lequel R₁, R₂, Y, Z et Q sont tels que définis précédemment pour le composé de formule (I) ; procédé dans lequel la chloration selon l'étape a) du procédé est effectuée selon un procédé en continu.

2. Procédé selon la revendication 1, dans lequel dans l'étape a) du procédé on utilise un solvant aprotique polaire.

3. Procédé selon la revendication 1, qui est effectué sans solvants.

4. Procédé selon l'une quelconque des revendications 1 à 3, qui est effectué en présence du 2-chloro-5-chlorométhylthiazole fabriqué précédemment.

5. Procédé de préparation d'un composé de formule 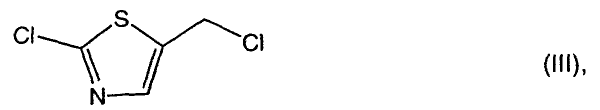 dans laquelle on fait réagir un composé de formule 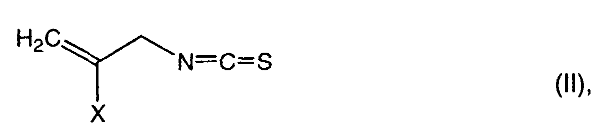 dans laquelle X est un groupe labile, sous forme libre ou sous forme de sel, avec un agent de chloration ; procédé dans lequel on effectue la réaction selon un procédé en continu.
